Europäisches Patentamt

European Patent Office

Office européen des brevets

(19)

(11) **EP 1 184 664 A1**

(12) # EUROPEAN PATENT APPLICATION

(43) Date of publication:
**06.03.2002 Bulletin 2002/10**

(51) Int Cl.[7]: **G01N 33/44**, G01N 31/16,
C08F 8/04, C08F 8/20

(21) Application number: **00114623.2**

(22) Date of filing: **07.07.2000**

(84) Designated Contracting States:
**AT BE CH CY DE DK ES FI FR GB GR IE IT LI LU MC NL PT SE**
Designated Extension States:
**AL LT LV MK RO SI**

(71) Applicant: **HONEYWELL B.V.**
**NL-1101 EA Amsterdam (NL)**

(72) Inventor: **Eggink, Roefof Geert**
**7681 CN Vroomshoop (NL)**

(74) Representative:
**Leson, Thomas Johannes Alois, Dipl.-Ing. et al**
**c/o TBK-Patent, P.O. Box 20 19 18**
**80019 München (DE)**

(54) **Method to determine the degree of halogenation or hydrogenation of gas valve rubber compounds**

(57) The invention relates to a method to determine the degree of halogination or hydrogenation of gas valve rubber compounds, especially of gas valve closing elements made from a rubber compound.

According to the invention a potassium permanganate solution is provided and the concentration of said potassium permanganate solution is determined by titration. Said potassium permanganate solution reacts with a halogenated or hydrogenated rubber compound, especially with the surface of said gas valve closing elements, and after a predetermined time the concentration of said potassium permanganate solution is determined by titration. The difference between these concentrations is determined and is a measure for the degree of halogination or hydrogenation.

## Description

The invention relates to a method or process to determine the degree of halogination or hydrogenation of the surface of a rubber closing element for a gas valve.

Gas valves usually comprise a closing element, made of a rubber compound, whereby said closing element acts against a valve seat made of metal or plastic. For a faultless operation of gas valves, the following aspects are of importance: In case of a closed gas valve the rubber compound of the closing element and the metal or plastic valve seat have to provide a sealed interaction. This means that the rubber compound should have a high sealing level. In case of a heat demand the gas valve has to open immediately and as a consequence the rubber compound of the closing element should have a low sticking level to the metal or plastic valve seat.

To provide a rubber compound for a closing element of a gas valve fulfilling the above-mentioned requirements, the closing elements undergo a special treatment to prevent stickiness onto the valve seat. The closing elements made of rubber are treated with a special halogen mixture or with hydrogen. This is done to cross-link the surface of the closing elements. The increase of the cross-link density of the surface of the closing element decreases the stickiness of the closing elements onto the valve seats to an acceptable level.

Up to now, there are only a few time consuming methods for the determination of the effects of the halogen or hydrogen treatment on the properties of the closing elements, e.g. special corrosion tests with closing elements and valve seats etc.

It is therefore an object of the present invention to provide a simple method to determine the degree of halogination or hydrogenation of gas valve rubber compounds.

The method according to the invention comprises the features of claim 1. Preferred embodiments can be taken from the description and the depended claims.

Rubber compounds for the closing element of a gas valve known from the prior art usually comprise the following ingredients:

- a base NBR elastomer, e.g. a Perbunan NT (available from the company Bayer)
- a vulcanisation systems, e.g. Sulphur Rhenocure S (available from the company Rhein Chemie)
- an activator, e.g. zinc oxide (available from the company Rhein Chemie)
- fillers, e.g. carbon black and/or silica
- plasticizers, e.g. Mesamoll (available from the company Bayer)
- anti oxidants.

The closing elements made of rubber are treated with a special halogen mixture or with hydrogen. This is done to cross-link the surface of the closing elements. The increase of the cross-link density of the surface of the closing element decreases the stickiness of the closing elements onto the valve seats to an acceptable level.

In order to determine the degree of halogination or hydrogenation of the surface of a rubber closing element for a gas valves, the closing elements are immersed in potassium permanganate solution for at least 30 min. The decrease in concentration of the potassium permanganate solution is determined by a simple titration, and the amount of reactive places in the rubber surface is determined. The difference in the amount of reactive places in the surface of non-halogenated or non-hydrogenated closing elements and halogenated or hydrogenated closing elements is an indication for the amount of halogen or hydrogen that reacted with the rubber surface.

The chemical determination is:

Closing elements are immersed in a 0,1 N potassium permanganate solution for at least 30 minutes.

The reactive bonds in the closing element surfaces will react with the potassium permanganate solution. After 30 min, the potassium permanganate solution is decanted and poured into a burette.

Approximately 2,250 grams of oxalic acid ($H_2C_2O_4.2\ H_2O$) is weighed up to the nearest 0,01 mg (= A mg) in an Erlenmeyer flask and 100 ml pure water ( 27 °C +/- 3 °C) and 100 ml sulfuric acid (2 M) are added to the Erlenmeyer flask. The oxalic acid should dissolve completely. This solution is called aqueous solution.

From the burette, approximately 35 ml of the said potassium permanganate solution is added to said aqueous solution of the Erlenmeyer flask. The pink color will disappear, and the resulting solution is heated to a temperature of 55 °C. The solution is then titrated to the moment that a weak and faint pink color is visible for at least 30 sec. At that moment, the exact amount of potassium permanganate solution (poured into the Erlenmeyer flask) is recorded (= V ml).

It is strongly recommended to repeat the same test with a mixture of only 100 ml sulfuric acid and 140 ml pure water. The amount of potassium permanganate from this titration is recorded as V0 ml.

The concentration of the potassium permanganate solution is calculated as:

$$t = (A/ (62,99^* (V - V0))).$$

The same measurement is executed on the potassium permanganate solution that came not in contact with closing elements. The difference in potassium permanganate concentration of the two solutions (with and without contact with closing elements) is a measure for the amount of reactive places in the surface of the closing elements. This corresponds to the determine the degree of halogination or hydrogenation. The difference in the amount of reactive places in the surface of non-halogenated or non-hydrogenated and of halogenated or hydrogenated closing elements is a measure for the amount of halogen or hydrogen , bonded to the surface of the closing elements.

The above-cited chemical determination is only a preferred embodiment of the invention. The chemical determination can vary as follows:

-   concentration of said potassium permanganate solution: 0,01 N to 1N, preferred: 0,1 N
-   said duration of treatment within said potassium permanganate solution: 15 min to 45 min, preferred: 30 min
-   said aqueous solution contains oxalic acid: 0,225 grams to 22,50 grams, preferred: 2,250 grams
-   said aqueous solution contains pure water 50 ml to 150 ml, preferred 100 ml / 140 ml
-   said temperature of said pure water: 24 °C to 30 °C , preferred: 27 °C
-   said aqueous solution contains sulfuric acid: 50 ml to 150 ml,
-   preferred: 100 ml / 100 ml
-   said amount of the said potassium permanganate solution to be added to said aqueous solution: 15 ml to 70 ml, preferred: 35 ml
-   said determination of V0 can be changed in such a way that the volume of water in this test equals the total volume of reactants in the determination of the concentration of potassium permanganate solutions.
-   said temperature of the solution: 50 °C to 60°C, preferred: 55 °C

**Claims**

1.  Method to determine the degree of halogination or hydrogenation of gas valve rubber compounds, especially of gas valve closing elements made from a rubber compound, wherein:

    a) a potassium permanganate solution is provided and the concentration of said potassium permanganate solution is determined by titration,

    b) said potassium permanganate solution reacts with a halogenated or hydrogenated rubber compound, especially with the surface of said gas valve closing elements, and after a predetermined time the concentration of said potassium permanganate solution is determined by titration,

    c) the difference between the potassium permanganate concentrations of steps a) and b) is determined.

2.  Method according to claim 1, wherein said predetermined time is in range from 15 min to 45 min, especially 30 min.

3.  Method according to claim 1 or 2, wherein after said predetermined time the potassium permanganate solution is decanted and mixed with an aqueous solution, wherein the resulting solution is heated to a predetermined temperature, wherein said solution is then titrated and the concentration of said potassium permanganate solution is determined.

4.  Method according to claim 3, wherein said aqueous solution is a mixture of pure water, sulfuric acid and oxalic acid.

5.  Method according to claim 3 or 4, wherein the resulting solution is heated to a temperature of 50 °C to 60 °C, especially 55 °C.

# EP 1 184 664 A1

**European Patent Office**

## EUROPEAN SEARCH REPORT

Application Number

EP 00 11 4623

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 4 631 309 A (THOERMER JOACHIM ET AL) 23 December 1986 (1986-12-23) * column 3, line 62 - column 4, line 16 * | 1-5 | G01N33/44 G01N31/16 C08F8/04 C08F8/20 |
| Y | EP 0 093 580 A (OCCIDENTAL CHEM CO) 9 November 1983 (1983-11-09) * page 7, line 18 - line 26 * | 1-5 | |
| A | DATABASE WPI Week 199506 Derwent Publications Ltd., London, GB; AN 1995-041408 '06! XP002156176 & JP 06 322161 A (INOAC CORP KK, KURIMOTO IRON WORKS LTD), 22 November 1994 (1994-11-22) * abstract * | 1 | |
| A | US 4 368 280 A (YUI HIROSHI ET AL) 11 January 1983 (1983-01-11) * column 11, line 50 - column 12, line 11 * | 1-5 | **TECHNICAL FIELDS SEARCHED** (Int.Cl.7) G01N C08F |
| A | DATABASE WPI Week 199410 Derwent Publications Ltd., London, GB; AN 1994-077329 '10! XP002156177 & JP 06 016892 A (NIPPONDENSO CO LTD), 25 January 1994 (1994-01-25) * abstract * | 1 | |
| A | US 4 839 429 A (TAJIMA MASAO) 13 June 1989 (1989-06-13) * tables 4,7,11 * | 1 | |

The present search report has been drawn up for all claims

| Place of search | Date of completion of the search | Examiner |
|---|---|---|
| THE HAGUE | 21 December 2000 | Bosma, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

4

**ANNEX TO THE EUROPEAN SEARCH REPORT
ON EUROPEAN PATENT APPLICATION NO.**              EP 00 11 4623

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2000

| Patent document cited in search report | | Publication date | Patent family member(s) | | Publication date |
|---|---|---|---|---|---|
| US 4631309 | A | 23-12-1986 | DE | 3226081 C | 15-12-1983 |
| | | | GB | 2123837 A,B | 08-02-1984 |
| EP 0093580 | A | 09-11-1983 | CA | 1231726 A | 19-01-1988 |
| | | | DE | 3368269 D | 22-01-1987 |
| | | | JP | 1631798 C | 26-12-1991 |
| | | | JP | 2056363 B | 30-11-1990 |
| | | | JP | 58225107 A | 27-12-1983 |
| JP 6322161 | A | 22-11-1994 | JP | 2739278 B | 15-04-1998 |
| US 4368280 | A | 11-01-1983 | NONE | | |
| JP 6016892 | A | 25-01-1994 | NONE | | |
| US 4839429 | A | 13-06-1989 | US | 4833206 A | 23-05-1989 |
| | | | US | 5082875 A | 21-01-1992 |

EPO FORM P0459